(19) **Europäisches Patentamt**
**European Patent Office**
**Office européen des brevets**

(11) **EP 3 128 939 B1**

(12) **EUROPEAN PATENT SPECIFICATION**

(45) Date of publication and mention
of the grant of the patent:
**19.06.2019 Bulletin 2019/25**

(21) Application number: **15711226.9**

(22) Date of filing: **23.03.2015**

(51) Int Cl.:
**A61B 34/00** (2016.01)

(86) International application number:
**PCT/EP2015/056128**

(87) International publication number:
**WO 2015/144640 (01.10.2015 Gazette 2015/39)**

(54) **ELECTROMAGNETIC NAVIGATION SYSTEM FOR MICROSCOPIC SURGERY**

ELEKTROMAGNETISCHES NAVIGATIONSSYSTEM FÜR MIKROSKOPISCHE CHIRURGIE

SYSTÈME DE NAVIGATION ÉLECTROMAGNÉTIQUE POUR CHIRURGIE MICROSCOPIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.03.2014 GB 201405227**

(43) Date of publication of application:
**15.02.2017 Bulletin 2017/07**

(73) Proprietor: **Scopis GmbH**
**10179 Berlin (DE)**

(72) Inventors:
• **WINNE, Christian**
**10179 Berlin (DE)**
• **REUTTER, Andreas**
**10179 Berlin (DE)**
• **KOSMECKI, Bartosz**
**10179 Berlin (DE)**
• **OZBEK, Christopher**
**10179 Berlin (DE)**

(74) Representative: **Röthinger, Rainer et al**
**Wuesthoff & Wuesthoff**
**Patentanwälte PartG mbB**
**Schweigerstrasse 2**
**81541 München (DE)**

(56) References cited:
**WO-A1-2005/067807     WO-A1-2007/113719
WO-A1-2013/013718     US-A1- 2008 147 086
US-A1- 2011 015 518     US-B2- 7 471 202**

## Description

FIELD OF THE INVENTION

The present invention relates to an electromagnetic navigation system for microscopic surgery and a method for correction of navigation data during microscopic surgery using said system.

BACKGROUND OF THE INVENTION

Navigation systems support during surgical interventions the surgeon in his orientation in the operating field. For that purpose, the position of the instruments used by the surgeon is visualized in a 3D image data of the patient. Continuous detection of the position and orientation (i.e. pose) of the patient and navigated tools (so-called navigation data) are necessary for this visualization using measuring systems with spatial accuracy of less than 1 mm. The computer-assisted imaging also enables in addition to the presentation of the navigation data, the selection and planning of relevant anatomical areas (so-called planning data) and displaying the navigation data with respect to this.

Only optical and electromagnetic measurement systems have been proven to be suitable as measuring systems for surgical navigation and represent the state of the art in this field. The optical measuring systems consist of stereo camera systems (so-called navigation cameras), which detect the 3D positions of the assembly of optical ball markers (e.g. active LED markers or retro reflective marker spheres), which are attached to the patient and the instruments. In contrast, an electromagnetic measuring system consists of a field generator for generating an electromagnetic field and small sensor coils. These coils are used to measure the field strength of the generated electromagnetic field and are attached to the patient as well as they are integrated into the navigated instruments. So far, the field generators are mounted on the side or under the operating table using articulated arms or they are completely integrated into the operating table.

Navigation systems are used in interventions under direct visual control of the surgeon, as well as in operations with the aid of additional optical viewing equipment. Here, surgical microscopes play an important role in surgery of sensitive and finely textured anatomical areas such as the central nervous system, the auditory system, the brain or the sinuses.

During microscopic interventions, the surgeon usually positions the microscope optics with the eyepiece above the surgical field with the aid of a microscope stand. The distance to the situs is dependent on the region of focus of the respective surgical microscope, but is usually between 200 to 500 mm. The physician looks through the eyepiece in direction of the operating field and works freehand due to the self-retaining microscope system.

The combination of microscopy with clinical navigation enables an overlay of navigation and planning data in the microscope image (also called 'Augmented Reality'). For example, it is possible to display tumour borders in the eyepiece, which can be regarded as established state of the art in neurosurgical procedures. However, it is necessary to know the intrinsic imaging properties and the current spatial position of the microscope in relation to the patient and used instruments, to facilitate augmented reality.

Document US4722056 discloses a method of integrating image information from an imaging device and an operating microscope during an operative procedure on body parts of a patient comprising a positioning of an operating microscope in the course of said operative procedure at an operative location relative to the patient, establishing the spatial relationship among the image information, the patient, and the focal plane of the microscope, introducing the image information and spatial relationship to a computer and reformatting the image information to generate a computer-generated image of the body part at a determined plane related to the focal plane of the microscope and projecting the computer-generated image into the optics of the operating microscope for coordinated viewing of the computer-generated image and the patient. A disadvantage of this method is that the only image information from the focal plane of the microscope is shown in the microscope view. Also, the described system uses an acoustic tracking system with acoustic transducers and microphones for determining the pose of the microscope. These acoustic tracking devices does not achieve the accuracy and practicability of current optical and electromagnetic tracking systems. In addition, no movement of the patient relative to the microphones of the tracking system is allowed.

Document DE000010249025B4 discloses a method for determining the 3D position of an instrument on the basis of markings arranged close to the tip of the instrument, analysis of the microscopic image and determination of the position in the z-direction, which is realized by distance analysis using depth-of-field-evaluation or stereoscopic image analysis. A disadvantage of this method is that the tracked instruments must be visible in the microscopic image.

Document EP1272862B1 discloses a process for detecting distortions to probe location or orientation determinations, comprising measuring a plurality of magnetic field values, the measured values depending on a probe's location and a probe's orientation, determining one of the probe's location and the probe's orientation from an extremum of an optimization function, the function depending on differences between the measured field values and field values from a model, determining one of the probe's location and the probe's orientation includes, guessing a location and orientation of the probe, calculating magnetic field values associated with the guessed location and orientation from a model and evaluating a new value of the optimization function from the calculated and measured values; rejecting the guessed location and orientation in response to the new value being further from an extremum of the function than an earlier calculated value of the function, indicating that a distortion to the determining exists in response to the extremum belonging to a preselected range of values associated with presence of distortion, wherein the act of indicating is responsive to presence of a passive distortion to the measured magnetic field by one of a nearby conductor and a ferromagnetic object, and wherein the act of indicating is responsive to presence of an active distortion to the measured magnetic field by a nearby field source. It is a disadvantage of this process that no special detection and correction of microscope depending errors, especially no detection of errors, which depend on the microscope settings are intended.

Document EP1613213B1 discloses a method of compensating for distortions due to the presence of conductive objects in the vicinity of a magnetic tracking system comprising determining an undisturbed phase $(\varphi_U)$ for at least one of a first position indication signal and a second position indication signal determining an undisturbed amplitude ratio that relates the amplitude $(A_U)$ of the first position indication signal at a first frequency to the amplitude $(A_U)$ of the second position indication signal at a second frequency, determining a disturbed amplitude $(A_T)$ and phase $(\varphi_T)$ of a position indication signal of at least one sensor and adjusting the position indication signal of said at least one sensor to compensate for distortions due to eddy currents, based on the disturbed amplitude $(A_T)$ and phase $(\varphi_T)$, the undisturbed amplitude ratio, and the undisturbed phase $((\varphi_U)$. It is a disadvantage that no special detection and correction of microscope depending errors, especially no detection of errors, which depend on the microscope settings are intended.

Document EP1303771B1 discloses a method for determining the position of a sensor element with the aid of which a magnetic alternating field emitted by at least one generator unit is measured, the position of the sensor element being determined on the basis of a signal received in the sensor element, further, preferably in first approximation, disturbing fields being calculated which result from eddy currents generated in electric conductive objects, and the position which is determinable from the signal received in the sensor element, being corrected on the basis of the calculated disturbing fields, wherein the eddy currents in the object are calculated on a basis of the alternating field, and that the disturbing fields are calculated on a basis of the calculated eddy currents. Again, it is a disadvantage of the disclosed method that no special detection and correction of microscope depending errors, especially no detection of errors, which depend on the microscope settings are intended.

Document EP1392151B1 discloses an electromagnetic navigation system for use in navigating a probe through an electromagnetic field positioned near a metal object, said electromagnetic navigation system comprising a transmitter coil array having a plurality of transmitter coils, said transmitter coil array operable to generate the electromagnetic field to navigate the probe, and a shield positioned adjacent the metal object that includes a fluoroscope, said shield formed of a material operable to shield the metal object from the electromagnetic field generated by said transmitter coil array, wherein said shield substantially reduces distortion of the electromagnetic field by the metal object characterized in that said transmitter coil array is attached to said shield. The shield is positioned on and optimized for a fluoroscope so that the disclosed system is restricted to the use of a fluoroscope. No special detection and correction of microscope depending errors, especially no detection of errors, which depends on the microscope settings are intended, which is another disadvantage.

Birkfellner et al. (Transactions on Medical Imaging, Vol. 17, No. (5), pages 737-742, October 1998) describe the combination of an optical and an electromagnetic tracking system in order to reach a better availability of tracking data during surgery. Therefore, the two tracking systems are registered to each other using an optical tracker and an electromagnetic sensor fixed at the navigated tool. The deviation in position between the electromagnetic sensor and the optical tracking system was measured in a standard operating room configuration, wherein the field generator was integrated into the operating table. One major disadvantage of the approach of this disclosure for microscopic surgery is the need of two tracking systems, which have to be installed in the operating room. Another disadvantage is the problem of pose measurement of the microscope by the electromagnetic tracking system due to its limited measurement volume.

Kindratenko (Virtual Reality: Research, Development, and Applications, Vol. 5., No. 3, pages 169-182, 2000) discloses a survey of various techniques used to calibrate electromagnetic tracking systems. These techniques allow the correction of static errors caused by metal or ferromagnetic material in the measurement field under the assumption that the position of the field generator is fixed and the surrounding metal does not move. This document does not provide a solution for calibrating electromagnetic tracking systems in a surgical environment, particularly with surgical microscopes in the vicinity of the field generator.

US2007/0244666A1 discloses a method for refining position und orientation measurement data of a tracked sensor coils during electromagnetic tracking. A discretized numerical field model is used to compute the refinement of position and orientation data especially in case of a distortion of the electromagnetic field (distortion mapping). The use of such a numerical field model aims at an improved stability of the distortion correction however the creation of a numerical field model requires a previous complex calibration based on a sensor data measurement at given positions in the measurement field in presence of the sources of electromagnetic field distortion e.g. using a robot arm.

GB2436707A discloses a medical electromagnetic tracking system also for microscope image-guided procedures which comprises electromagnetic receivers (sensor coils) that are attached to the to-be-tracked instruments and to the surgical microscope. To handle tracking errors caused by the electromagnetic field distortion of the microscope, the approach of distortion correction and calibration was mentioned. Alternatively the mounting of the sensor coil in a larger distance to the microscope is described. Nevertheless, the large distance of the microscope to the patient and to the field generator is still a problem. The attachment of the transmitter coils (field generator) at the skull of the patient as described is usually not an option due to the weight and size of the field generator.

Thus, in microscopic surgery, the following disadvantages of known navigation systems can be summarized:

• Optical navigation systems suffer from a blocked line of sight, resulting in the use of microscopes above the operating field and the cooperation of several surgeons. The positions of instruments and the patient can thus not be measured easily by an optical measurement system.

• The surgical microscope as a metallic and ferromagnetic object near the field generator can lead to significant malfunctions and errors of the electromagnetic position measurement. These disturbances of the electromagnetic field, induced by the microscope, can more importantly depend on the position of the field generator relative to the microscope and on the microscope settings (e.g. zoom and focus).

• The measuring range of the electromagnetic navigation system is too small to capture the poses of the sensor coil of the patient as well as of the instruments and of the microscope.

Currently, no satisfactory solutions for integrating an EM measurement system for microscopic procedures are known. The following problems are related to known solutions:

• EM sensor coils must be attached to the microscope. The position determination of EM sensors on the microscope is disturbed by the metal of the microscope, potentially no measurement of the pose of the microscope is possible

• EM field sizes are usually too small to detect simultaneously, reliably and robust the patient, the instruments and the microscope

BRIEF SUMMARY OF THE INVENTION

The present invention provides an electromagnetic navigation system according to claim 1. Further embodiments of the invention can be gathered from the dependent claims. An electromagnetic navigation system for microscopic surgery is provided, comprising at least a microscope and an electromagnetic measurement system comprising a field generator, wherein the field generator is fixed or adjustably connected to the microscope, and at least one sensor element with sensor coils for measuring the pose of navigated instruments and/or the patient; and an disturbance correction module for compensating effects of disturbances of the electromagnetic field caused by the microscope.

It is intended that the field generator can be arranged at the bottom of the microscope and may be connected by an adjustment element to the microscope.

The microscope of an electromagnetic navigation system according to the present disclosure may have at least one of a video output interface, an image injection module and a settings communication interface.

It is envisaged for the sensor element electromagnetic navigation that it might be a patient tracker or pointer.

The electromagnetic navigation system according to the present disclosure may further comprise a data acquisition unit for receiving the current microscope images, microscope settings and the current navigation data.

It is further intended that a data storage unit in which a three-dimensional image data set of the object area containing the object volume are storable may be a component of an electromagnetic navigation system of the present disclosure.

It is envisaged that the electromagnetic navigation system of the present disclosure may further comprise a data processing unit with an image data registering module with which the pose of the three-dimensional image data set relative to the field generator can be determined, a microscope registration module with which the intrinsic imaging properties and the pose of the microscope optics can be determined relative to the field generator for various zoom and focus settings of the microscope and , if applicable, for the current state of the adjustment element, based on a previous calibration or constructive parameters of the microscope and an image computation module for generation at least one of the following views / image data, with virtual two-dimensional image data with the positional correct overlay of planning data in the microscope image and / or for image injection into the microscope optics, slice images of the three-dimensional image data with optionally visualized planning data.

It is further intended that the electromagnetic navigation system of the present disclosure may further comprise an image display unit with which computed image data or views can be displayed.

A physical shielding is placed between surgical microscope and field generator, fixed to the microscope and / or the field generator, in an electromagnetic navigation system according to the present disclosure.

Another object of the present invention is a method for the correction of navigation data during microscopic surgery using an electromagnetic navigation system, comprising the steps of calibrating an electromagnetic measurement system for determining a mapping rule for the calculation of corrected poses of sensor elements to compensate for the effects of the disturbance of the electromagnetic field by the microscope and/or the shielding based on pose data of the sensor elements in a disturbance-free environment; and use of mapping rule to correct the poses data of the sensor elements during surgery.

It is intended for the method that the mapping rule uses distortion mapping, wherein polynomials or splines are used for calculating corrected poses of the sensor elements.

The electromagnetic sensors may be rigidly coupled to the microscope to detect changes of the electromagnetic field relative to the state of calibration.

It is further envisaged that the disturbance correction module uses data obtained during microscopic surgery for compensating disturbances of the electromagnetic measurement system during surgery.

Another object of the present invention is the use of an electromagnetic navigation system in microscopic surgery, comprising at least a microscope; an electromagnetic measurement system comprising a field generator, wherein the field generator is fixed or adjustably connected to the microscope, at least one sensor element with sensor coils for measuring the pose of navigated instruments and/or the patient; and an disturbance correction module for compensating effects of disturbances of the electromagnetic field caused by the microscope

BRIEF DESCRIPTION OF THE FIGURES

The present invention will be described by figures and examples. It is obvious for a person ordinary skilled in the art that the scope of the invention is not limited to the disclosed embodiments. It shows:

Figure 1   System setup with microscope and electromagnetic measuring system

Figure 2   Possible embodiment of the field generator and its fixed mounting with shielding on the microscope, the field generator is arranged in a ring around the microscope lens.

Figure 3   Further possible embodiment of the field generator and its adjustable mounting with shielding on the microscope

Figure 4   Embodiment of figure 3 with indicated visual field of the microscope and measuring range of electromagnetic measurement system

Figure 5   Data flow between the system components

DETAILED DESCRIPTION OF THE INVENTION

The present invention provides an electromagnetic navigation system for microscopic surgery, comprising at least a microscope; and an electromagnetic measurement system comprising a field generator, wherein the field generator is fixed or adjustably connected to the microscope, and a sensor with one or more sensor coils; and an additional disturbance correction module for compensating disturbances of the electromagnetic measurement system caused by the microscope

The terms 'shield' or 'shielding' will be used synonymously within the meaning of the present disclosure. The position and spatial orientation of a sensor for instance will be designated as the 'pose' of the respective sensor, so that pose summarizes position and spatial orientation within the meaning of the present disclosure. A 'sensor element' summarizes all elements bearing at least one sensor coil so that the pose of the sensor element within the electromagnetic field can be determined. A sensor element can be integrated into a pointer instrument, into a patient tracker or into any other element which might be used during surgery and wherein it is necessary to know the correct position of such a sensor element.

The following components may be part of the system:

- Surgical microscope with optional video output interface, with an optional image injection unit and an optional settings communication interface;
- Electromagnetic measurement system, consisting of a field generator, patient trackers and instruments, characterized in that the field generator is fixed or adjustably connected by an adjustment element to the operating microscope, and characterized by an additional disturbance correction module;
- Data acquisition unit for receiving the current navigation data and optionally the current microscope images and microscope settings as well as the state of the adjustment element;
- Data storage unit in which a three-dimensional image data set of the object area containing the object volume is storable;
- Data processing unit with:

  - an image data registering module with which the pose of the three-dimensional image data set relative to the field generator can be determined
  - a microscope registration module with which the intrinsic imaging properties and the pose of the microscope optics can be determined relative to the field generator for various zoom and focus settings of the microscope, if applicable, based on a previous calibration or constructive parameters of the microscope
  - Image computation module for generation of the following views / image data:

- Virtual two-dimensional image data with the positionally correct overlay of planning data in the microscope image and / or for image injection into the microscope optics
- Slice images of the three-dimensional image data with optionally visualized planning data

- Image display unit for displaying computed image data or views
- optional: Physical shielding between surgical microscope and field generator, fixed to microscope and / or field generator

A surgical microscope is predominantly used in minimally invasive surgery and microsurgery. It is mounted on a floor or a ceiling support and is positioned and oriented above the patient by the surgeon. The microscope provides a magnified stereoscopic view of the operating field. The surgeon sees the image, which is captured by the microscope lens, through the two eyepieces of the microscope. The microscope image, perceived by the surgeon, is additionally optionally captured with the aid of photo sensors and provided via digital or analog video interfaces.

Some surgical microscopes have additional data interfaces for the exchanging the current microscope settings such as zoom or focus. Additionally, the possibility to include additional image data in the microscopic optics using beam splitters may be given (so-called image injection).

An electromagnetic measuring system consists of a field generator in which a number of coils are installed in different orientations. These coils generate an alternating electromagnetic field. Further components are one or more small sensor coils located within the sensor, in which a current is induced by the electromagnetic field. By measuring the induced current, the measurement system can uniquely determine the position of the sensor coil, respectively the sensor. These coils are used as part of patient trackers and tools to measure the poses of patients and instruments. The measuring systems are calibrated previously for a disturbance free environment. Ferromagnetic material near the field generator changes the electromagnetic field and thus influences the position detection of the sensor coils.

The disturbance of the measuring system caused by the microscope and/or by a shielding has to be optimally compensated in order to allow a correct position measurement of the patient and the used navigated instruments.

One possible implementation is "distortion mapping correction". With that, a correction for the position and orientation of the sensor is calculated based on a previous calibration for each position in the measurement field. The position and orientation of the sensor coil can be described as a translation (X, Y, Z) and orientation in the form of Euler angles ($\Psi$, $\Theta$, $\Phi$). The correction can be also be dependent of the current microscope settings (microscope parameter vector $\mathbf{m}$) and the current setting of the field generator mounting adjustment element (parameter vector $\mathbf{f}$). Hence the correction function yields for the calculation of the corrected pose vector $\mathbf{p}_c$ based on the input parameters $\mathbf{q}=(x, y, z, \Psi, \Theta, \Phi, \mathbf{m}, \mathbf{f})$:

$$\mathbf{p}_c = \begin{pmatrix} x_c \\ y_c \\ z_c \\ \Psi_c \\ \Theta_c \\ \Phi_c \end{pmatrix} = g(\mathbf{q}) = g \begin{pmatrix} x \\ y \\ z \\ \Psi \\ \Theta \\ \Phi \\ \mathbf{m} \\ \mathbf{f} \end{pmatrix}$$

Possible implementations of the correction function g can be based on polynomials or splines. With polynomials the corrected output values $\mathbf{p}_c = (x_c, y_c, z_c, \Psi_c, \Theta_c, \Phi_c)^T$ based on the input parameters $\mathbf{q}=(x, y, z, \Psi, \Theta, \Phi, \mathbf{m}, \mathbf{f})$ with the single values $\mathbf{q}_i$ and $1 <= i <= n$ are determined as follows:

$$\mathbf{p}_c = \begin{pmatrix} x_c \\ y_c \\ z_c \\ \Psi_c \\ \Theta_c \\ \Phi_c \end{pmatrix} = \mathbf{A} \begin{pmatrix} 1 \\ q_1^1 \\ q_1^2 \\ \vdots \\ q_1^k \\ q_2^1 \\ q_2^2 \\ \vdots \\ q_2^k \\ \vdots \\ q_n^1 \\ q_n^2 \\ \vdots \\ q_n^k \end{pmatrix}$$

wherein k indicates the degree of the polynomials and A indicates a $6 \times (n \cdot k+1)$ matrix which contains the coefficients of the polygons that are determined during an initial calibration.

By using a known shielding, the influence on the electromagnetic field can be calculated using the finite difference method or the finite element method. In particular, the change of direction and strength of the field in the measurement space of the electromagnetic measuring system can be quantitatively predicted. This allows in large parts to compensate the influence of the electromagnetic field caused by the shielding.

The data acquisition unit captures, digitizes and if necessary temporarily stores the data, which is continuously arriving from the microscope image data stream, and the communicated microscope settings for zoom and focus. Furthermore, the position and orientation data of the sensor coils are cached, detected by the electromagnetic measuring system, and made available to the data processing module.

The data storage unit stores the three-dimensional image data of the patient and, if necessary the calibration data of the disturbance correction module of the electromagnetic measuring system and of the microscope registration module. These data are made available when needed for the data processing unit.

The data processing unit has the task to combine navigation data from the electromagnetic measuring system, the 3D image data and calibration data from the data storage unit and the microscope images from the surgical microscope, in order to calculate useful views and images for the user. They may include:

- Slice images of the 3-D image data at the position of the tool center point of a navigated instrument or the focal point of the microscope

- Perspective or orthographic 3-D view of the image data of the patients with illustrated position of the tracked instruments and/or the microscope

- Microscope image view with positionally correct superposition of planning data based on the pre-operative 3-D image data and the navigation data

- Virtual microscope image with planning data for injecting in the microscope op-tic.

The following sub-modules may be part of the data processing unit:

The image data registration module has the task of determining continuously the spatial relationship between the field generator of the electromagnetic measuring system and the 3D image data set. For this purpose, an electromagnetic sensor is usually attached to the patient near the operating field in order to serve as a reference system for the position of the 3D image data. The pose of this electromagnetic sensor is continuously detected by the electromagnetic measuring system. Methods for the intraoperative determination of the rigid transformation between the reference coordinate systems of the 3D image data and of the patient sensor are well-known in state of the art, such as landmarks-based and surface-based methods (Eggers, Mühling & Marmulla, International Journal of Oral and Maxillofacial Surgery, 2006, 35(12), 1081-1095.; Simon, Hebert & Kanade, International Journal of Oral and Maxillofacial Surgery, 1995, 35(12), 1081-1095), which can be used in this module.

The microscope registration module calculates and continuously updates the imaging properties of the microscope as a function of the current microscope settings (e.g. zoom and focus) and, if applicable, of the setting of the adjustment element of the field generator. When modeling the microscope as a pinhole camera, the intrinsic and extrinsic camera parameters for the current zoom and focus setting of the microscope can be determined based on a previous calibration. The intrinsic parameters may include the focal length, the image center and the aspect ratio and parameters for the description of non-linear distortion. The extrinsic camera parameters include the translational and rotational parameters of the rigid transformation between the reference coordinate system of the electromagnetic measuring system and the origin of the pinhole camera model.

The computation of the camera parameters can be calculated similarly to the disturbance correction module using polynomials or splines based on the current zoom and focus settings of the microscope or on the setting of the adjustment element. Relevant prior art in this field of microscope registration is (Edwards et al., IEEE Transactions on Medical Imaging, 2000, 19(11), 1082-1093; Garcia Girladez et al., International Journal of Computer Assisted Radiology and Surgery, 2007, 1(5), 253-264; Holloway, 1995, University of North Carolina at Chapel Hill, Chapel Hill, NC, USA). The microscope registration is computed automatically during surgery, a support by the user is not necessary.

The image computation module calculates views and image data for the presentation on the image display unit or for the injection in the microscope optic. Here, the current output data of the image data registration module, of the microscope registration module and of the data acquisition unit are used, in particular the current transformation between the electromagnetic measuring system and 3D image data and other navigated instruments, the current transformation between the electromagnetic measuring system and the origin of the camera system in accordance with the pinhole camera model and the intrinsic camera parameters.

These transformations allow transferring the patient planning data marked in the 3D image data into the reference coordinate system of the microscope camera. Then, the current intrinsic camera parameters are used to calculate the mapping of the planning data onto the image plane of the microscope. This enables the computation of the virtual microscope images. For the superimposed microscope image views, the real and virtual microscope image data are combined and shown on the image display unit.

In addition, these transformations also allow the calculation of slice images of the 3D image data through the position of the tool center point of the navigated instrument or through the focal point of the microscope.

The image display unit is used to display the generated views and image data on a screen.

The optional shielding is made of a ferromagnetic material, which disturbs the electromagnetic field of the electromagnetic measuring system, and thus overlays the disturbance caused the microscope or other metal objects. The knowledge of the disturbance of the electromagnetic field by the shielding is gained during a simulation and/or a calibration. So, the influencing of the navigation data of the measuring system can be eliminated or at least substantially reduced due to the shielding.

The shielding can preferably have the following properties:

- The shield can consists of ferromagnetic material, in particular aluminum.

- The shielding is mounted between the microscope and field generator.

- The shielding is flat, or consists of one or more flat plates, in order to achieve a sufficient disturbance overlay in particular of the microscope.

- The shielding may have an opening at the position of the microscope lens in order not to interrupt the view of the microscope on the operation field.

The following system modifications can be of advantage:

Sensor coils can be mounted or installed rigidly to the field generators. During surgery, the pose of the electromagnetic sensors can be continuously monitored and checked for changes. Firstly, this helps to determine whether at least one field generator, the microscope or the shielding are not in the expected configuration. Secondly, a temporary disturbance of the electromagnetic field caused by an unexpected adjustment of zoom and focus of the microscope by electric motors can be detected. The detection of these disturbances allows the warning of the user and the prevention of the display of incorrect position information.

Another modification is to have a dynamically adjustable position and orientation of the field generator under the microscope. The following benefits are expected:

- Optimization of the average volume of the viewing area of the microscope and the working space of the electromagnetic measuring system

- Automatic optimal alignment of the electromagnetic measuring system, both on the field of view of the microscope and on the position of the patient reference sensor, which can be mounted distantly.

The improved integration of the electromagnetic measuring system is realized by a rigid, possibly reproducible mounting of the field generator at the microscope, particularly at the bottom of the microscope. This allows an initial calibration of the pose and of the imaging characteristics of the microscope optical system relative to a reference coordinate system of the electromagnetic measuring system. For a correct overlay of planning data in the microscope image no intraoperative calibration will be necessary.

To compensate the disturbance which is expected to be caused by the metal of the surgical microscope, a disturbance correction module is introduced which eliminates or reduced the influencing of the surgical microscope based on a one-time-only calibration of the changes of the electromagnetic field. To minimize the disturbance, which is caused by the metal of the surgical microscope, a shielding can be mounted between the field generator and the microscope. In that case, the shielding overlays the disturbance of the microscope and minimizes the effects of the microscope disturbances on the pose measurement.

The advantages of the invention are:

- usability of electromagnetic navigation for microscopic surgery

- No time-consuming alignment of the field generator to the operation area as the surgeon performs the alignment implicitly by positioning the microscope

- No intraoperative calibration is necessary as the position and orientation of the field generator is known relative to the microscope optics

DETAILED DESCRIPTION OF THE FIGURES

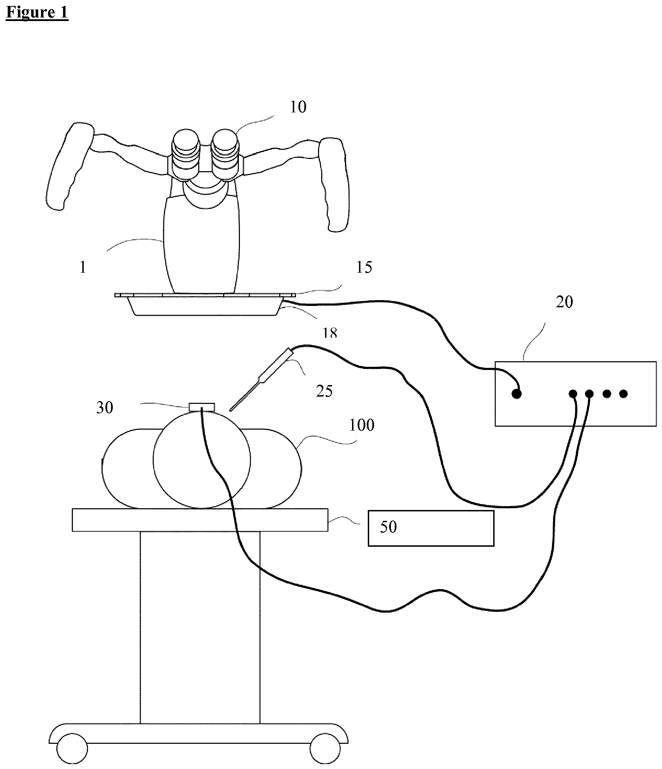

Figure 1 shows a system structure with an operating microscope, the electromagnetic (EM) measurement system consisting of the field generator and the EM sensors, which are integrated into the navigated instruments and the patient tracker. The field generator is mounted below the microscope and creates an electromagnetic field for measuring the position of EM sensors with integrated sensor coils underneath the microscope.

Between the microscope and the field generator is a shield in the form of a plate, particularly made of ferromagnetic material. The field generator and the EM sensors in the navigated instruments and the patient trackers are connected via cables to the EM-measuring system. During surgery, the microscope is positioned over the surgical field where the patient is positioned generally on the operating table. The surgeon sees the highly magnified stereoscopic view of the operating field through the eyepieces of the microscope.

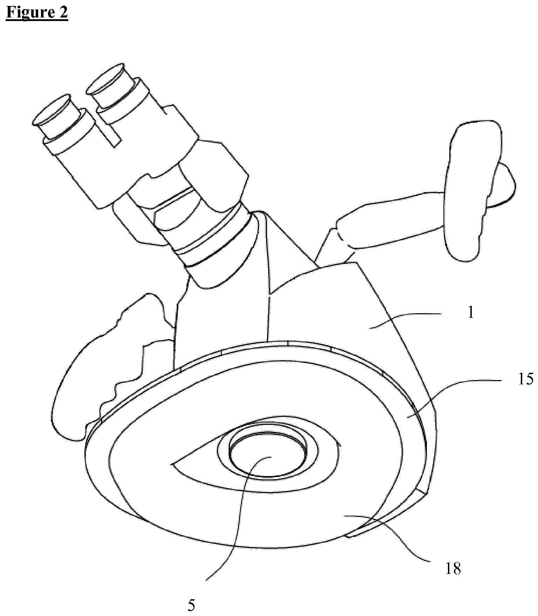

Figure 2 discloses an embodiment, wherein the field generator is fixed mounted to the microscope together with a shield. The field generator is constructed with an opening in its centre, so that an uninterrupted view of the microscope through the microscope lens on the operating field is possible. The shield has a corresponding circular opening, if necessary an additional annular shield around this opening must be attached. The shape of the field generator is approximately circular, but the shape is optimized for the installation of elongated coils. Alternative forms such a torus or other solid of revolutions are also conceivable.

The size and shape of the field generator is configured such that the measuring range of the electromagnetic measurement system covers at least the sharply displayable field of view of the microscope.

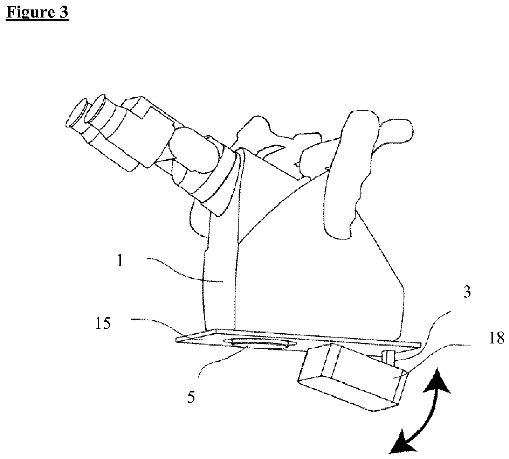

Figure 3 shows a further possible embodiment of the field generator and its attachment to the microscope. Here, the field generator is approximately cuboid and is mounted below the microscope beside the microscope lens. With the aid of an adjusting element, the orientation of the field generator can be changed manually or automatically towards the microscope lens so that the measurement range of the electromagnetic measurement system include as much as possible of the field of view of the microscope.

The adjusting element is realized as in this embodiment as a linear actuator in combination with a rotational joint. Alternative implementations of the mounting of the field generator on the microscope can be realised with rotational or spherical joints, which are driven pneumatically or hydraulically if necessary. The adjustment element also comprises a sensor element to detect the state of the adjustment element and to enable to deduce the current position of the field generator relative to the microscope. Further, a shield is mounted between the microscope and the field generator, which has an opening for the microscope lens.

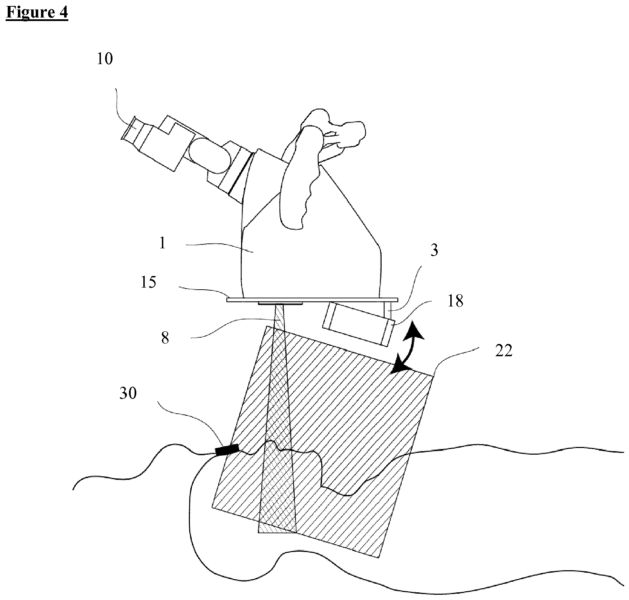

Figure 4 shows the same embodiment as depicted in figure 3. In addition, the field of view of the microscope and the measuring range of the electromagnetic measurement system are shown in the hatched areas. It is obvious that the orientation of the field generator under the microscope is crucial for achieving an overlap of the microscopic field of view and the measurement range of the electromagnetic measurement system. The overlapping area is necessary for the pose measurement of EM sensors or navigated instruments, which are visible in the microscope image. An additional requirement arising from the need for continuous measurement of the pose of the patient tracker is to locate the patient tracker in the measurement region of the electromagnetic measuring system. To ensure this, the orientation of the field generator can be changed intra-operatively manually or automatically, if necessary. Optimising the orientation of the field generator so that the patient tracker and the navigated instruments are located in the optimum measurement region of the electromagnetic measurement system can also increase the accuracy of the position measurement.

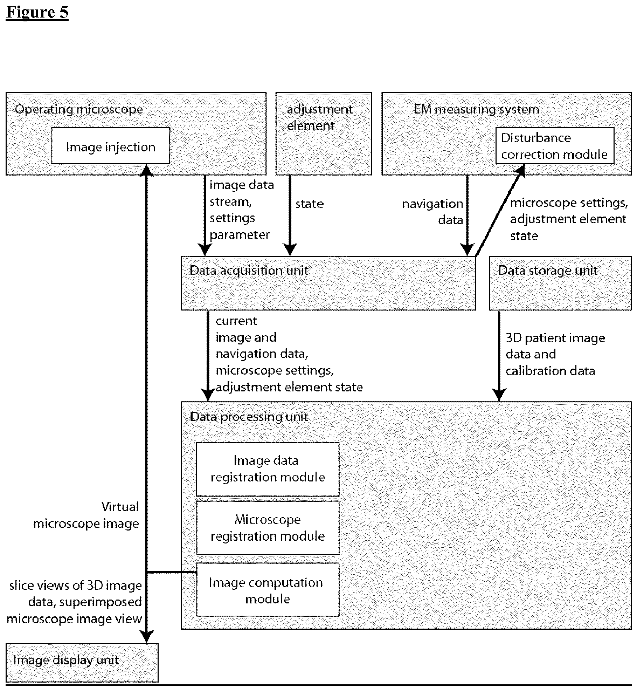

Figure 5 discloses the data flow between the system components and subcomponents for the system configuration with an operating microscope, which supports image injection. The main components include the surgical microscope, the electromagnetic measurement system, the adjustment element, the data acquisition unit, the data storage unit, the data processing unit and the image display unit.

The microscope provides a continuous data stream of the current microscope image data using a digital or analogue video interface. This video data stream as well as a separate data stream with the current microscope settings, such as zoom and focus is transferred to the data acquisition unit. Additionally, the electromagnetic measurement system transmits the navigation data as well as the adjustment element its state to the data acquisition unit.

The data acquisition unit receives the microscope settings data and the adjustment element state. Those data are transmitted to the disturbance correction module in order to perform a correction of the navigation data dependent on the current microscope settings and mounting of the field generator on the microscope. The data acquisition unit provides the data processing module with all current data of the microscope and the measurement system. In addition, the data processing unit can access the 3D image data of the patient and calibration data via the data storage unit. The subcomponents image data registration modules, microscope registration module and the image computation module process this data. The image computation module calculates image data for display on the image display unit as well as for injecting in the microscope optics.

REFERENCE NUMBER LIST

| | |
|---|---|
| 1 | Microscope |
| 3 | Adjustment Element |
| 5 | Microscope Lens |
| 8 | Microscopic Field of View |
| 10 | Eyepiece |
| 15 | Shielding |
| 18 | Field Generator |
| 20 | Electromagnetic Measurement System |
| 22 | Electromagnetic Measurement Region |
| 25 | Pointer with sensor elements |
| 30 | Patient Tracker with sensor elements |
| 50 | Operating Table |
| 100 | Patient |

**Claims**

1. An electromagnetic navigation system for use with a navigated instrument (25) to perform microscopic surgery on a patient (100), the system comprising at least:

    a. a surgical microscope (1);
    b. an electromagnetic measurement system (20) comprising a field generator (18), wherein the field generator (18) is fixed or adjustably connected to the surgical microscope (1);

c. a shielding (15) between the surgical microscope (1) and the field generator (18), fixed to the surgical microscope (1) and/or the field generator (18);

d. at least one sensor element with sensor coils for measuring the pose of the navigated instruments (25) and/or the patient (100); and

e. a disturbance correction module for compensating effects of disturbances of the electromagnetic field caused by the surgical microscope (1) and/or the shielding (15).

2. The electromagnetic navigation system of claim 1, wherein the field generator (18) is arranged at the bottom of the surgical microscope (1) and creates an electromagnetic field for measuring the pose of the at least one sensor element underneath the surgical microscope (1).

3. The electromagnetic navigation system of claim 1, wherein the surgical microscope (1) further comprises a microscope lens (5); and wherein the shielding (15) has an opening at the position of the microscope lens (5) in order not to interrupt the view of the surgical microscope (1) on the operation field.

4. The electromagnetic navigation system of claim 1 or 2, wherein the field generator (18) is connected to the surgical microscope (1) by an adjustment element (3) configured to manipulate the orientation of the field generator (18) relative to the surgical microscope (1).

5. The electromagnetic navigation system of one of claims 1 to 4, wherein the at least one sensor element is part of a patient tracker (30) or a pointer (25).

6. The electromagnetic navigation system of one of claims 1 to 5, further comprising a data acquisition unit for receiving the current microscope images, microscope settings and the current navigation data.

7. The electromagnetic navigation system of one of claims 1 to 6, further comprising a data storage unit in which a three-dimensional image data set of the object area containing the object volume is storable.

8. The electromagnetic navigation system of one of claims 1 to 7, further comprising a data processing unit with:

a. an image data registering module with which the pose of the three-dimensional image data set relative to the field generator (18) can be determined;

b. a microscope registration module with which the intrinsic imaging properties and the pose of the microscope optics can be determined relative to the field generator (18) for various zoom and focus settings of the microscope and, if applicable, for the current state of the adjustment element (3), based on a previous calibration or constructive parameters of the surgical microscope (1); and

c. an image computation module for generating at least one of the following views or image data, with:

i. virtual two-dimensional image data with the positionally correct overlay of planning data in the microscope image and/or for image injection into the microscope optics; and/or

ii. slice images of the three-dimensional image data with optionally visualized planning data.

9. The electromagnetic navigation system of one of claims 1 to 8, further comprising an image display unit with which computed image data or views can be displayed.

10. A method for correction of navigation data during microscopic surgery using an electromagnetic navigation system of any of claims 1 to 9, comprising the steps of:

a. calibrating an electromagnetic measurement system for determining a mapping rule for the calculation of corrected pose of an EM sensor to compensate for the effects of the disturbance of the electromagnetic field by the surgical microscope (1) and/or the shielding (15) based on pose data of the at least one sensor element (25, 30) in a disturbance-free environment; and

b. use of the mapping rule to correct the pose data of the EM sensor during surgery.

11. The method of claim 10, wherein the electromagnetic navigation system comprises an adjustment element (3) for adjusting intra-operatively the orientation of the field generator (18) relative to the surgical microscope (1) to achieve overlap of the microscopic field of view (8) and the measurement range of the electromagnetic measurement system (22).

12. The method of claim 10, wherein the mapping rule uses distortion mapping, wherein polynomials or splines are used for calculating corrected pose of the at least one sensor element (25, 30).

13. The method of claim 10, wherein an electromagnetic sensor is rigidly coupled to the surgical microscope (1) to detect changes of the electromagnetic field relative to the state of calibration.

**Patentansprüche**

1. Elektromagnetisches Navigationssystem zur Verwendung mit einem navigierten Instrument (25), $\mu$m eine mikroskopische Operation an einem Patienten (100) durchzuführen, wobei das System zumindest umfasst:

   a. ein chirurgisches Mikroskop (1);
   b. ein elektromagnetisches Messsystem (20), das einen Feldgenerator (18) umfasst, wobei der Feldgenerator (18) fest oder einstellbar mit dem chirurgischen Mikroskop (1) verbunden ist;
   c. eine Abschirmung (15) zwischen dem chirurgischen Mikroskop (1) und dem Feldgenerator (18), die an dem chirurgischen Mikroskop (1) und/oder an dem Feldgenerator (18) befestigt ist;
   d. zumindest ein Sensorelement mit Sensorspulen zum Messen der Lage des navigierten Instruments (25) und/oder des Patienten (100); und
   e. ein Störungskorrekturmodul zum Kompensieren von Effekten von Störungen des elektromagnetischen Felds, die von dem chirurgischen Mikroskop (1) und/oder von der Abschirmung (15) verursacht werden.

2. Elektromagnetisches Navigationssystem nach Anspruch 1, wobei der Feldgenerator (18) an der Unterseite des chirurgischen Mikroskops (1) angeordnet ist und ein elektromagnetisches Feld zum Messen der Lage des zumindest einen Sensorelements unterhalb des chirurgischen Mikroskops (1) erzeugt.

3. Elektromagnetisches Navigationssystem nach Anspruch 1, wobei das chirurgische Mikroskop (1) ferner eine Mikroskoplinse (5) umfasst; und wobei die Abschirmung (15) eine Öffnung an der Position der Mikroskoplinse (5) aufweist, um die Sicht des chirurgischen Mikroskops (1) auf den Operationsbereich nicht zu unterbrechen.

4. Elektromagnetisches Navigationssystem nach Anspruch 1 oder 2, wobei der Feldgenerator (18) durch ein Einstellelement (3) mit dem chirurgischen Mikroskop (1) verbunden ist, das dazu eingerichtet ist, die Orientierung des Feldgenerators (18) relativ zu dem chirurgischen Mikroskop (1) zu beeinflussen.

5. Elektromagnetisches Navigationssystem nach einem der Ansprüche 1 bis 4, wobei das zumindest eine Sensorelement ein Teil eines Patienten-Trackers (30) oder eines Pointers (25) ist.

6. Elektromagnetisches Navigationssystem nach einem der Ansprüche 1 bis 5, ferner umfassend eine Datenerfassungseinheit zum Empfangen der gegenwärtigen Mikroskopbilder, Mikroskopeinstellungen und der gegenwärtigen Navigationsdaten.

7. Elektromagnetisches Navigationssystem nach einem der Ansprüche 1 bis 6, ferner umfassend eine Datenspeichereinheit, in der ein dreidimensionaler Bilddatensatz des Objektbereichs speicherbarer ist, der das Objektvolumen enthält.

8. Elektromagnetisches Navigationssystem nach einem der Ansprüche 1 bis 7, ferner umfassend eine Datenverarbeitungseinheit mit:

   a. einem Bilddaten-Registrierungsmodul, mit dem die Lage des dreidimensionalen Datensatzes relativ zu dem Feldgenerator (18) bestimmt werden kann;
   b. einem Mikroskop-Registrierungsmodul, mit dem die intrinsischen Bildgebungseigenschaften und die Lage der Mikroskopoptik relativ zu dem Feldgenerator (18) auf der Grundlage einer vorhergehenden Kalibrierung oder konstruktiver Parameter des chirurgischen Mikroskops (1) für verschiedene Vergrößerungs- und Fokuseinstellungen des Mikroskops und, falls anwendbar, für den gegenwärtigen Zustand des Einstellelements (3) bestimmt werden kann; und
   c. ein Bild-Berechnungsmodul zum Erzeugen zumindest einer der folgenden Ansichten oder Bilddaten, mit:

      i. virtuellen zweidimensionalen Bilddaten mit der auf die Position bezogen korrekten Überlagerung von Planungsdaten in dem Mikroskopbild und/oder zur Bildeinbringung in die Mikroskopoptik hinein; und/oder
      ii. Schichtbildern der dreidimensionalen Bilddaten mit optional visualisierten Planungsdaten.

9. Elektromagnetisches Navigationssystem nach einem der Ansprüche 1 bis 8, ferner umfassend eine Bildanzeigeeinheit, mit der berechnete Bilddaten oder Ansichten angezeigt werden können.

10. Verfahren zum Korrigieren von Navigationsdaten während einer mikroskopischen Operation unter Verwendung eines elektromagnetischen Navigationssystems nach einem der Ansprüche 1 bis 9, umfassend die Schritte:

   a. Kalibrieren eines elektromagnetischen Messsystems zum Bestimmen einer Kartierungsregel für die Berechnung einer korrigierten Lage eines EM-Sensors, um die Effekte der Störung des elektromagnetischen Felds durch das chirurgische Mikroskop (1) und/oder die Abschirmung (15) auf der Grundlage von Lagedaten des zumindest einen Sensorelements (25, 30) in einer störungsfreien Umgebung auszugleichen; und

b. Verwenden der Kartierungsregel, um die Lagedaten des EM-Sensors während der Operation zu korrigieren.

11. Verfahren nach Anspruch 10, wobei das elektromagnetische Navigationssystem ein Einstellelement (3) zum intra-operativen Einstellen der Orientierung des Feldgenerators (18) relativ zu dem chirurgischen Mikroskop (1) umfasst, um das mikroskopische Sichtfeld (8) und den Messbereich des elektromagnetischen Messsystems (22) zu überlagern.

12. Verfahren nach Anspruch 10, wobei die Kartierungsregel eine Störungskartierung verwendet, wobei Polynome oder Splines zum Berechnen einer korrigierten Lage des zumindest einen Sensorelements (25, 30) verwendet werden.

13. Verfahren nach Anspruch 10, wobei ein elektromagnetischer Sensor mit dem chirurgischen Mikroskop (1) starr verbunden ist, um Änderungen des elektromagnetischen Felds relativ zu dem Kalibrationszustand zu erkennen.

**Revendications**

1. Système de navigation électromagnétique pour une utilisation avec un instrument navigué (25) pour exécuter une chirurgie microscopique sur un patient (100), le système comprenant au moins :

   a. un microscope chirurgical (1) ;
   b. un système de mesure électromagnétique (20) comprenant un générateur de champ (18), dans lequel le générateur de champ (18) est fixé ou connecté de façon ajustable au microscope chirurgical (1) ;
   c. un blindage (15) entre le microscope chirurgical (1) et le générateur de champ (18), fixé au microscope chirurgical (1) et/ou au générateur de champ (18) ;
   d. au moins un élément de capteur avec des bobines de capteur pour mesurer la posture des instruments navigués (25) et/ou du patient (100) ; et
   e. un module de correction de perturbations pour compenser des effets de perturbations du champ électromagnétique causées par le microscope chirurgical (1) et/ou le blindage (15).

2. Système de navigation électromagnétique selon la revendication 1, dans lequel le générateur de champ (18) est agencé au fond du microscope chirurgical (1) et crée un champ électromagnétique pour mesurer la posture de l'au moins un élément de capteur en-dessous du microscope chirurgical (1).

3. Système de navigation électromagnétique selon la revendication 1, dans lequel le microscope chirurgical (1) comprend en outre un objectif (5) de microscope ; et

dans lequel le blindage (15) a une ouverture à la position de l'objectif (5) de microscope afin de ne pas interrompre le vue du microscope chirurgical (1) sur le champ d'opération.

4. Système de navigation électromagnétique selon la revendication 1 ou 2, dans lequel le générateur de champ (18) est connecté au microscope chirurgical (1) par un élément d'ajustement (3) configuré pour manipuler l'orientation du générateur de champ (18) par rapport au microscope chirurgical (1).

5. Système de navigation électromagnétique selon l'une des revendications 1 à 4, dans lequel l'au moins un élément de capteur fait partie d'un traqueur (30) de patient ou d'un pointeur (25).

6. Système de navigation électromagnétique selon l'une des revendications 1 à 5, comprenant en outre une unité d'acquisition de données pour recevoir les images de microscope courantes, les réglages de microscope et les données de navigation courantes.

7. Système de navigation électromagnétique selon l'une des revendications 1 à 6, comprenant en outre une unité de stockage de données dans laquelle un ensemble de données d'images tridimensionnelles de la zone objet contenant le volume objet peut être stocké.

8. Système de navigation électromagnétique selon l'une des revendications 1 à 7, comprenant en outre une unité de traitement de données avec :

   a. un module d'enregistrement de données d'images avec lequel la posture de l'ensemble de données d'images tridimensionnelles par rapport au générateur de champ (18) peut être déterminée ;
   b. un module d'enregistrement de microscope avec lequel les propriétés intrinsèques d'imagerie et la posture de l'optique de microscope peuvent être déterminées par rapport au générateur de champ (18) pour divers réglages de zoom et de focalisation du microscope et, si applicable, pour l'état courant de l'élément d'ajustement (3), sur la base d'un étalonnage antérieur ou de paramètres de construction du microscope chirurgical (1) ; et
   c. un module de calcul d'images pour générer au moins une des vues ou des données d'images suivantes, avec :

> i. des données d'images bidimensionnelles virtuelles avec la superposition positionnellement correcte de données de planification dans l'image de microscope et/ou pour une injection d'image dans l'optique de microscope ; et/ou
> ii. des images de tranches des données d'images tridimensionnelles avec des données de planification facultativement visualisées.

9. Système de navigation électromagnétique selon l'une des revendications 1 à 8, comprenant en outre une unité d'affichage d'images avec laquelle des données d'images calculées ou des vues peuvent être affichées.

10. Procédé de correction de données de navigation lors d'une chirurgie microscopique utilisant un système de navigation électromagnétique selon l'une quelconque des revendications 1 à 9, comprenant les étapes de :

> a. étalonnage d'un système de mesure électromagnétique pour déterminer une règle de mappage pour le calcul d'une posture corrigée d'un capteur EM pour compenser les effets de la perturbation du champ électromagnétique par le microscope chirurgical (1) et/ou le blindage (15) sur la base de données de posture de l'au moins un élément de capteur (25, 30) dans un environnement dépourvu de perturbations ; et
> b. utilisation de la règle de mappage pour corriger les données de posture du capteur EM lors de la chirurgie.

11. Procédé selon la revendication 10, dans lequel le système de navigation électromagnétique comprend un élément d'ajustement (3) pour ajuster intra-opérationnellement l'orientation du générateur de champ (18) par rapport au microscope chirurgical (1) pour réaliser un chevauchement du champ de vision microscopique (8) et de la plage de mesure du système de mesure électromagnétique (22).

12. Procédé selon la revendication 10, dans lequel la règle de mappage utilise un mappage de distorsion, dans lequel des polynômes ou des splines sont utilisés pour calculer une posture corrigée de l'au moins un élément de capteur (25, 30).

13. Procédé selon la revendication 10, dans lequel un capteur électromagnétique est couplé de façon rigide au microscope chirurgical (1) pour détecter des changements du champ électromagnétique par rapport à l'état d'étalonnage.

**Figure 1**

**Figure 2**

**Figure 3**

**Figure 4**

**Figure 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4722056 A
- DE 000010249025 B4
- EP 1272862 B1
- EP 1613213 B1
- EP 1303771 B1
- EP 1392151 B1
- US 20070244666 A1
- GB 2436707 A

**Non-patent literature cited in the description**

- **BIRKFELLNER et al.** *Transactions on Medical Imaging,* October 1998, vol. 17 (5), 737-742
- *Virtual Reality: Research, Development, and Applications,* 2000, vol. 5 (3), 169-182
- **EGGERS ; MÜHLING ; MARMULLA.** *International Journal of Oral and Maxillofacial Surgery,* 2006, vol. 35 (12), 1081-1095
- **SIMON ; HEBERT ; KANADE.** *International Journal of Oral and Maxillofacial Surgery,* 1995, vol. 35 (12), 1081-1095
- **EDWARDS et al.** *IEEE Transactions on Medical Imaging,* 2000, vol. 19 (11), 1082-1093
- **GARCIA GIRLADEZ et al.** *International Journal of Computer Assisted Radiology and Surgery,* 2007, vol. 1 (5), 253-264
- Holloway. University of North Carolina at Chapel Hill, 1995